# EUROPEAN PATENT SPECIFICATION

(11) **EP 2 543 262 B1**
(45) Date of publication and mention of the grant of the patent: **18.02.2015**
(21) Application number: 12174854.5
(22) Date of filing: 03.07.2012
(51) Int. Cl.: A23L 1/226, A61Q 11/00, C07C 233/09, A61K 8/42, A23G 4/06

(54) **Flavor-enhancing amide compounds**
Geschmacksverstärkende Amidverbindungen
Composés d'amide à amélioration de la saveur

(30) Priority: 08.07.2011 US 201113178550
(43) Date of publication of application: 09.01.2013
(73) Proprietor: INTERNATIONAL FLAVORS & FRAGRANCES INC., New York New York 10019 (US)
(72) Inventor: Janczuk, Adam Jan, New Jersey, NJ08857 (US); Liu, Zhihua, New Jersey, NJ 08816 (US)
(74) Representative: Lawrence, John

(56) References cited:
- US-A1- 2006 057 268
- US-A1- 2007 134 389

## Description

### Field of the Invention

The present invention relates to novel amide compounds and their incorporation and use to enhance or modify the flavor of orally consumable compositions, such as foodstuff, chewing gums, dental and oral hygiene products, and medicinal products.

### Background of the Invention

The term umami, from the Japanese word to describe savory or meaty, is a term used to describe the unique overall fullness and savory taste of food. Materials that exhibit this taste quality generally potentate the intensity of glutamate solutions, which is an important characteristic of the umami taste. Umami is increasingly becoming recognized as the fifth sense of taste, the others being sour, sweet, salt, and bitter. Compounds traditionally described as possessing the umami character are monosodium glutamate (MSG), protein hydrolysates, some amino acids, certain nucleotides, and phosphates.

MSG is the most widely used material as a "taste enhancer" where it synergizes the perception of "savory" ingredients. However, a large amount of MSG may cause adverse effect as well as allergic reactions in human.

Other chemical compounds such as certain nucleotides also exhibit the umami effect, which include adenosine 5'-(trihydrogen diphosphate) (ADP), 5'-cytidylic acid (CMP), 5'-uridylic acid (UMP), 5'-adenylic acid (AMP), 5'-guanylic acid (GMP), 5'-inosinic acid (IMP), 5'-guanylic acid disodium salt, and 5'-inosinic acid disodium salt. Recent literature cites an extensive range of other organic compounds as taste active components of mixtures shown to provide the umami effect. These compounds include but are not necessarily limited to: organic acids such as succinic acid, lactic acid, saturated straight chain aliphatic acids of six, eight, fourteen, fifteen, sixteen, and seventeen carbon chain lengths, Z4,Z7,Z10,Z13,Z16,Z19-docosahexaenoic acid, ZS,Z8,Z11,Z14,Z17-eicosapentaenoic acid, Z9,Z12,Z16,Z19-octadecadienoic acid, Z9-octadecenoic acid, glutaric acid, adipic acid, suberic acid, and malonic acid. Amino acids having umami effect reported in the literature include glutamic acid, aspartic acid, threonine, alanine, valine, histidine, proline, tyrosine, cystine, methionine, pyroglutamic acid, leucine, lycine, and glycine. Dipeptides possessing umami properties include Val-Glu and Glu-Asp. Other miscellaneous compounds having umami properties include alpha-amino adipic acid, malic acid, alpha-aminobutyric acid, alpha-aminoisobutyric acid, E2,E4-hexadienal, E2,E4-heptadienal, E2,E4-octadienal, E2,E4-decadienal, Z4-heptenal, E2,Z6-nonadienal, methional, E3,E5-octadien-2-one, 1,6-hexanediamine, tetramethylpyrazine, trimethylpyrazine, cis-6-dodecen-4-olide, glutamate glycoconjugates, fish sauce blended with anchovy paste (U.S. Patent 7,510,738) and a number of naturally occurring amino acids.

Thus, despite the previous disclosure in the art, there is an ongoing need for novel flavor compounds, particularly those that enhance or modify the umami flavor, preferably lower the level of MSG in various food products to provide advantageous properties as well as economy of use.

### Summary of the Invention

The present invention provides novel amide compounds represented by Formula I set forth below and their unexpected and advantageous flavor enhancement and modification properties:

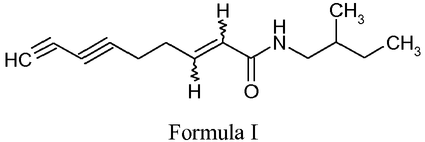

Specifically, the following isomeric compounds are disclosed:

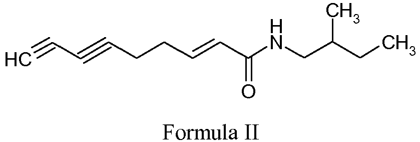

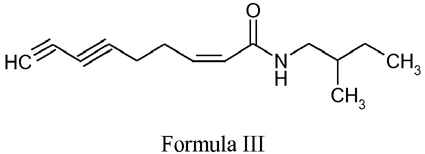

Another embodiment of the present invention relates to a process of augmenting, enhancing or imparting a taste to a material selected from the group consisting of foodstuff, a chewing gum, a dental or oral hygiene product, and a medicinal product comprising the step of incorporating an olfactory effective amount of the compound as defined above and an umami compound.

Another embodiment of the invention relates to a composition comprising the compound as defined above and an umami compound.

These and other embodiments of the present invention will be apparent by reading the following specification.

### Detailed Description of the Invention

It is known to those with the skill in the art that Formulas I as defined above provides the following isomeric compounds:

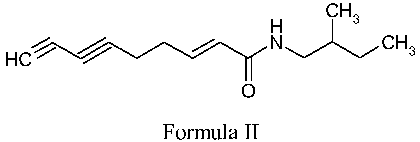

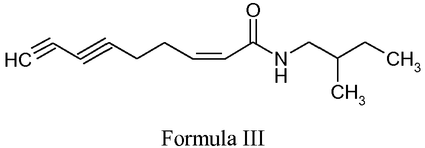

Those with the skill in the art will recognize that
Formula II represents (E)-N-(2-methylbutyl)nona-2-en-6,8-diynamide; and
Formula III represents (Z)-N-(2-methylbutyl)nona-2-en-6,8-diynamide.

The compounds of the present invention can be prepared from pent-4-yn-1-ol (commercially available from Sigma-Aldrich, Inc.). The reaction steps can be depicted by general schemes shown as follows: 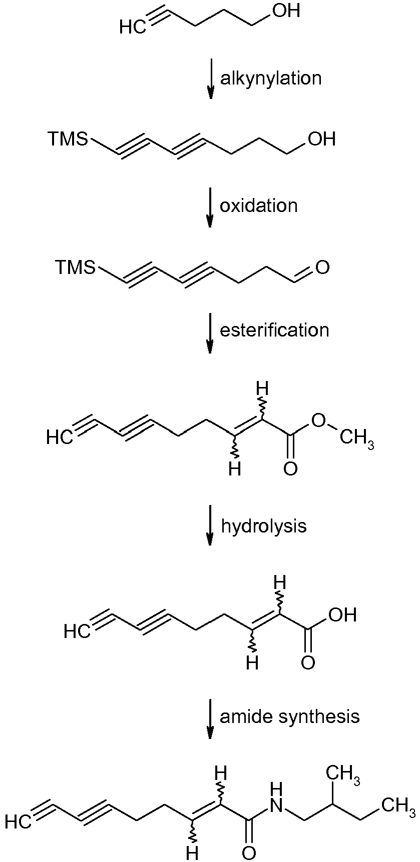 wherein TMS represents trimethylsilane.

The compounds of the present invention are surprisingly found to have unexpected properties of enhancing umami in flavors, which are demonstrated to be advantageous for the use in augmenting or imparting taste enhancement or somatosensory effect to foodstuff, chewing gums, oral hygiene products, and medicinal products by providing flavor enhancement and a preferred overall flavor profile.

The compounds of the present invention can be used in combination with other umami compounds known in the art. For example, the compounds may be employed to enhance the perceived umami taste of MSG since large amount of MSG may cause adverse effect as well as allergic reactions in human. In a preferred embodiment, the compounds are used in combination with MSG or a MSG-IMP mixture (2:1 by weight) in a weight ratio of at least about 1:20,000, preferably from about 1:2,000 to about 1:2, more preferably from about 1:1,000 to about 1:10, and even more preferably from about 1:500 to about 1:20.

As used herein, an umami compound is understood to mean a compound that exhibits umami flavor.

As used herein, an olfactory effective amount is understood to mean the amount of the compound in a flavor composition that alters the characteristics of the composition, or enhances or modifies the flavor, taste, and aroma reaction contributed by another ingredient in the composition. The overall flavor, taste, and aroma effect of the composition will be the sum effect of all flavor ingredients. The olfactory effective amount will vary depending on many factors including other ingredients, their relative amounts, and the effect that is desired.

The usage level of the compounds of the present invention varies depending on the product in which the compounds are employed. Generally, the level of the compounds employed in a product is greater than about 1 part per billion by weight, preferably from about 10 parts per billion to about 100 parts per million by weight, more preferably from about 50 parts per billion to about 10 parts per million by weight.

As used herein, foodstuff includes both solid and liquid ingestible materials for man or animals, which materials usually do, but need not, have nutritional value. Thus, foodstuff includes food products, such as meats, gravies, soups, convenience foods, malt, alcoholic and other beverages, milk and dairy products, seafood, including fish, crustaceans, mollusks and the like, candies, vegetables, cereals, soft drinks, snacks, dog and cat foods, other veterinary products and the like.

When the compounds of the present invention are used in an orally consumable composition, they can be combined with conventional flavoring ingredients or adjuvants, which are well known in the art. Requirements of such flavoring ingredients and adjuvants are that: (1) they be organoleptically compatible with the compounds of the present invention whereby the flavor of the ultimate consumable composition to which the compounds are added is not detrimentally affected by the use of such flavoring ingredients and adjuvants; and (2) they be ingestible acceptable and thus nontoxic or otherwise non-deleterious. In addition, the orally consumable composition can broadly include other flavor materials, vehicles, stabilizers, thickeners, surface active agents, conditioners, and flavor intensifiers.

The following are provided as specific embodiments of the present invention. Other modifications of this invention will be readily apparent to those skilled in the art. Such modifications are understood to be within the scope of this invention. As used herein all percentages are weight percent unless otherwise noted, ppb is understood to stand for parts per billion, ppm is understood to stand for parts per million, L is understood to be liter, mL is understood to be milliliter, g is understood to be gram, mol is understood to be mole, and mmol is understood to be millimole.

### EXAMPLE I

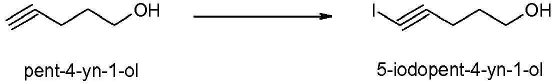

**Preparation of 5-Iodopent-4-yn-1-ol:** Potassium hydroxide (115 g, 2.051 mol, commercially available from Sigma-Aldrich, Inc.) was dissolved in water (150 mL) and cooled to 0 °C. Pent-4-yn-1-ol (69 g, 820 mmol, commercially available from Sigma-Aldrich, Inc.) was dissolved in methanol (1.125 L) and slowly added to the reaction mixture while the temperature was maintained at 0 °C. After 15-30 minutes, iodine (229 g, 902 mmol) was added in one portion and the mixture was warmed to room temperature and stirred for 3 hours. The mixture was then diluted with water (750 mL) and washed three times with diethyl ether (Et₂O) (300 mL). The organic layers were combined and concentrated *in vacuo* to provide yellow oil. The oil was dissolved in methylene chloride (CH₂Cl₂) (300 mL), washed with brine (300 mL), dried with sodium sulfate (Na₂SO₄), and filtered. The solvent was then removed *in vacuo* to provide the crude product (170 g), which was purified by column chromatography with hexane:ethyl acetate (Hex:EtOAc) to provide 5-iodopent-4-yn-1-ol (154 g, 89% yield).
¹H NMR (500 MHz, CDCl₃) δ: 1.50 ppm (s, 1H), 1.77 ppm (pentet, 2H, J = 6.50 Hz), 2.50 ppm (t, 2H, J = 6.92 Hz), 3.74 ppm (s, 2H). EIMS *mlz*: M⁺ 172.

### EXAMPLE II

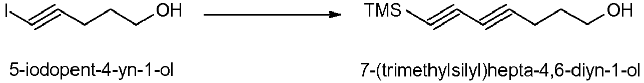

**Preparation of 7-(Trimethylsilyl)hepta-4,6-diyn-1-ol:** Ethynyltrimethylsilane (112 g, 1.143 mol, commercially available from Sigma-Aldrich, Inc.), piperidine (847 mL, 8.571 mol, commercially available from Sigma-Aldrich, Inc.), and 5-iodopent-4-yn-1-ol (120 g, 0.571 mol, prepared as above) were combined and cooled to 0 °C. Copper(I) chloride (CuCl) (5.66 g, 57.1 mmol, commercially available from Sigma-Aldrich, Inc.) was added in one portion. The reaction mixture was stirred with gradual warming to room temperature. After 30 minutes, the solution was quenched with saturated ammonium chloride solution (NH₄Cl) (2.5 L) and washed three times with Et₂O (300 mL). The organic layers were combined, washed twice with brine (500 mL), dried with Na₂SO₄, filtered, and concentrated *in vacuo* using a rotary evaporator. The crude product was purified by silica gel chromatography (Hex:EtOAc) (6:1) to provide 7-(trimethylsilyl)hepta-4,6-diyn-1-ol (91.8 g, 86% yield).
¹H NMR (500 MHz, CDCl₃) δ: 0.18 ppm (s, 9H), 1.53 ppm (s, 1H), 1.78 ppm (pentet, 2H, J = 6.56 Hz), 2.41 ppm (t, 2H, J = 6.97 Hz), 3.74 ppm (t, 2H, J = 5.88 Hz). EIMS *mlz*: M⁺ 180

### EXAMPLE III

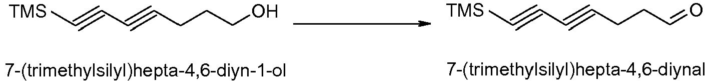

**Preparation of 7-(Trimethylsilyl)hepta-4,6-diynal:** Dimethyl sulfoxide (DMSO) (76 mL, 1.076 mol) was added dropwise at -78 °C to a solution of oxalyl chloride (47.1 mL, 538 mmol, commercially available from Sigma-Aldrich, Inc.) in CH₂Cl₂ (750 mL). The reaction mixture was stirred for 20 minutes at -78 °C. 7-(Trimethylsilyl)hepta-4,6-diyn-1-ol (50 g, 269 mmol, prepared as above) was dissolved in CH₂Cl₂ (15 mL) and slowly added. The reaction mixture was stirred for 1 hour at -78 °C. Triethylamine (Et₃N, 225 mL, 1.614 mol, commercially available from Sigma-Aldrich, Inc.) was then added. The reaction mixture was further stirred for 80 minutes while slowly warming to room temperature. The reaction mixture was quenched with saturated NH₄Cl, separated, and the aqueous portion was back-extracted twice with CH₂Cl₂ (200 mL). The organic layers were combined, dried with Na₂SO4, filtered, and concentrated *in vacuo.* The crude was purified by column chromatography (Hex:EtOAc) to provide 7-(trimethylsilyl)hepta-4,6-diynal (22.9 g, 47.8% yield).
¹H NMR (500 MHz, CDCl₃) δ: 0.18 ppm (s, 9H), 2.60 ppm (t, 2H, J = 7.07 Hz), 2.71 ppm (t, 2H, J = 7.09 Hz), 9.78 ppm (s, 1H). EIMS *mlz*: M⁺ 178

### EXAMPLE IV

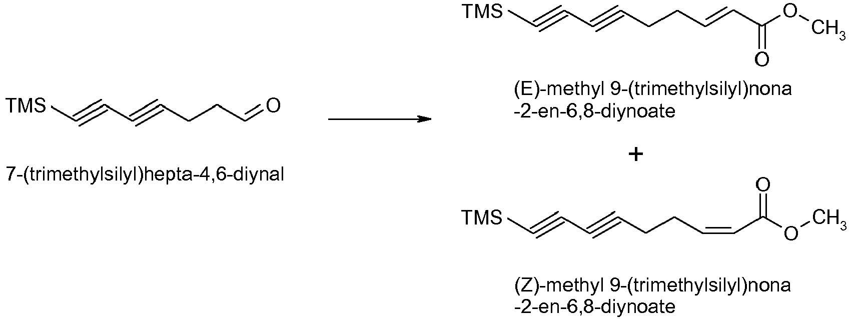

**Preparation of (E)-Methyl 9-(trimethylsilyl)nona-2-en-6,8-diynoate and (Z)-Methyl 9-(trimethylsilyl)nona-2-en-6,8-diynoate:** A solution of sodium hydride (5.89 g, 147 mmol) in tetrahydrofuran (THF) (500 mL) was cooled to 0 °C. Methyl 2-(dimethoxyphosphoryl)acetate (25.3 mL, 172 mmol, commercially available from Sigma-Aldrich, Inc.) was added dropwise and the reaction mixture was stirred for 20 minutes while the temperature was maintained at 0 °C. 7-(Trimethylsilyl)hepta-4,6-diynal (17.5 g, 98 mmol, prepared as above) was dissolved in THF (50 mL), added dropwise while gradually warming to room temperature, and stirred for 1 hour. The reaction mixture was quenched with saturated NH₄Cl (500 mL). The aqueous portion was extracted three times with Et₂O (200 mL). The organic layers were combined, dried with Na₂SO₄, filtered, and concentrated *in vacuo.* The crude product had an E:Z isomeric ratio of 8:1 and was purified by column chromatography (Hex:EtOAc) to provide the products (E)-methyl 9-(trimethylsilyl)nona-2-en-6,8-diynoate (16.0 g, 69.6% yield) and (Z)-methyl 9-(trimethylsilyl)nona-2-en-6,8-diynoate (2.0 g, 8.69% yield).

(E)-Methyl 9-(trimethylsilyl)nona-2-en-6,8-diynoate had the following NMR spectral characteristics:
¹H NMR (500 MHz, CDCl₃) δ: 0.19 ppm (s, 9H), 2.45 ppm (s, 4H), 3.74 ppm (s, 3H), 5.89 ppm (d, 1H, J = 15.57 Hz), 6.95 ppm (d, 1H, J = 15.67 Hz, of d, J = 6.18 Hz). MS *m*/*z*: M⁺ 234.

(Z)-Methyl 9-(trimethylsilyl)nona-2-en-6,8-diynoate had the following NMR spectral characteristics:
¹H NMR (500 MHz, CDCl₃) δ: 0.18 ppm (s, 9H), 2.44 ppm (t, 2H, J = 6.95 Hz), 2.88 ppm (q, 2H, J = 7.04 Hz), 3.71 ppm (s, 3H), 5.86 ppm (d, 1H, J = 11.46 Hz), 6.30 ppm (d, 1H, J = 11.41 Hz, of d, J = 7.28 Hz). EIMS *m*/*z*: M⁺ 234

### EXAMPLE V

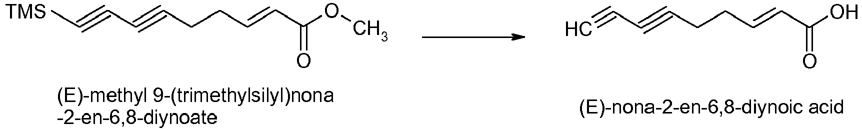

**Preparation of (E)-Nona-2-en-6,8-diynoic acid:** (E)-Methyl 9-(trimethylsilyl)nona-2-en-6,8-diynoate (8.4 g, 35.8 mmol, prepared as above) was added to a solution of sodium hydroxide (NaOH) (25%, 10 mL) and stirred at 40 °C. The reaction was monitored by thin layer chromatography (TLC) (Hex:EtOAc). After 24 hours at room temperature, the reaction mixture was neutralized to pH 6.5 and extracted with EtOAc. The organic layer was washed with water and brine, dried with magnesium sulfate (MgSO₄), and concentrated *in vacuo* to provide the product (E)-nona-2-en-6,8-diynoic acid as brown-white solid (4.8 g, 91% yield).
¹H NMR (500 MHz, CDCl₃) δ: 2.00 ppm (s, 1H), 2.43-2.51 ppm (m, 4H), 5.91 ppm (d, 1H, J = 15.65 Hz), 7.03-7.10 ppm (m, 1H). EIMS m/z: M⁺ 148

### EXAMPLE VI

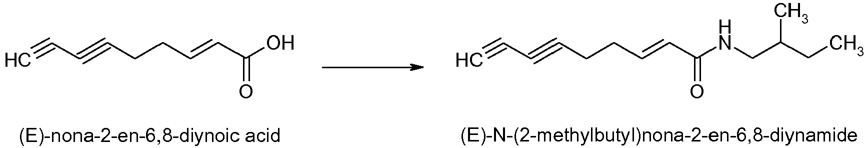

**Preparation of (E)-N-(2-Methylbutyl)nona-2-en-6,8-diynamide:** (E)-Nona-2-en-6,8-diynoic acid (0.5 g, 3.37 mmol, prepared as above), Et₃N (0.751 g, 7.42 mmol), N1-((ethylimino)methylene)-N3,N3-dimethylpropane-1,3-diamine hydrochloride (0.712 g, 3.71 mmol, commercially available from Sigma-Aldrich, Inc.), and 1H-benzo[d][1,2,3]triazol-1-ol hydrate (0.568 g, 3.71 mmol, commercially available from Sigma-Aldrich, Inc.) were dissolved in dimethylformamide (DMF) (20 mL). After 30 minutes, 2-methylbutan-1-amine (0.353 g, 4.05 mmol) dissolved in DMF (1 mL) was added dropwise to the reaction mixture. The reaction was monitored by TLC till completion. The reaction mixture was then poured over water and extracted with EtOAc (3 x 50 mL). The organic layers were combined and washed with water and brine, dried with MgSO₄, filtered and concentrated *in vacuo.* The crude product was purified by column chromatography (Hex:EtOAc) (3:1) to provide (E)-N-(2-methylbutyl)nona-2-en-6,8-diynamide (0.3 g, 40.9% yield).
¹H NMR (500 MHz, CDCl₃) δ: 0.91 ppm (d, 3H, J = 6.68 Hz), 0.91 ppm (t, 3H, J = 7.38 Hz), 1.12-1.27 ppm (m, 1H), 1.35-1.46 ppm (m, 1H), 1.55-1.60 ppm (m, 1H), 1.98 ppm (s, 1H), 2.42 ppm (s, 4H), 3.11-3.22 ppm (m, 1H), 3.25-3.31 ppm (m, 1H), 5.49 ppm (br. s, 1H), 5.84 ppm (d, 1H, J = 15.19 Hz), 6.77-6.83 ppm (m, 1H). EIMS *m*/*z*: M⁺ 217

### EXAMPLE VII

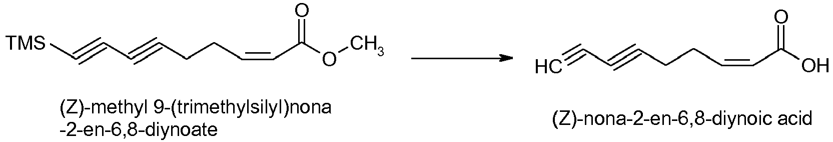

**Preparation of (Z)-Nona-2-en-6,8-diynoic acid:** (Z)-Methyl 9-(trimethylsilyl)nona-2-en-6,8-diynoate (2.8 g, 11.95 mmol, prepared as above) was added to NaOH solution (10%, 10 mL) and stirred at room temperature. The reaction was monitored by TLC (Hex:EtOAc). After 24 hours at room temperature, the reaction mixture was neutralized to pH 6.5 and extracted with EtOAc. The organic layer was washed with water and brine, dried with MgSO₄, and concentrated *in vacuo* to provide the product (Z)-nona-2-en-6,8-diynoic acid as brown-white solid (1.1 g, 62.1% yield).
¹H NMR (500 MHz, CDCl₃) δ: 1.99 ppm (s, 1H), 2.44 ppm (t, 2H, J = 7.01 Hz), 2.88-2.94 ppm (q, 2H, J = 7.11 Hz, of d, J = 1.54 Hz), 5.90 ppm (d, 1H, J = 11.49 Hz), 6.43 ppm (d, 1H, J = 11.49 Hz, of t, J = 7.31 Hz). EIMS *mlz*: M⁺ 148

### EXAMPLE VIII

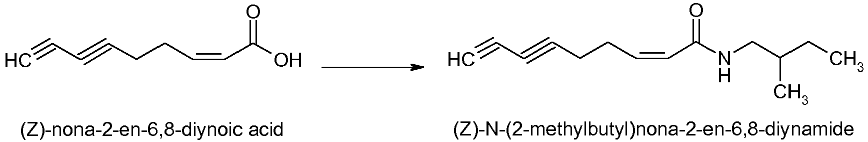

**Preparation of (Z)-N-(2-Methylbutyl)nona-2-en-6,8-diynamide:** (Z)-Nona-2-en-6,8-diynoic acid (0.5 g, 3.37 mmol, prepared as above), Et₃N (0.751 g, 7.42 mmol), N1-((ethylimino)methylene)-N3,N3-dimethylpropane-1,3-diamine hydrochloride (0.712 g, 3.71 mmol, commercially available from Sigma-Aldrich, Inc.), and 1H-benzo[d][1,2,3]triazol-1-ol hydrate (0.568 g, 3.71 mmol, commercially available from Sigma-Aldrich, Inc.) were dissolved in dimethylformamide (DMF) (20 mL). After 30 minutes, 2-methylbutan-1-amine (0.353 g, 4.05 mmol) dissolved in DMF (1 mL) was added dropwise. The reaction was monitored by TLC. When the reaction was complete, the reaction mixture was poured over water and extracted three times with EtOAc (50 mL). The organic layers were combined and washed with water and brine, dried with MgSO₄, filtered and concentrated *in vacuo.* The crude product was purified by column chromatography (Hex:EtOAc) (3:1) to provide (Z)-N-(2-methylbutyl)nona-2-en-6,8-diynamide (0.35 g, 47.7% yield).
¹H NMR (500 MHz, CDCl₃) δ: 0.91 ppm (d, 3H, J = 6.67 Hz), 0.91 ppm (t, 3H, J = 7.38 Hz), 1.12-1.22 ppm (m, 1H), 1.37-1.46 ppm (m, 1H), 1.53-1.62 ppm (m, 1H), 1.97 ppm (s, 1H), 2.43 ppm (t, 2H, J = 6.87 Hz), 2.89 ppm (q, 2H, J = 7.06 Hz), 3.06-3.13 ppm (m, 1H), 3.21-3.27 ppm (m, 1H), 5.63 ppm (br. s, 1H), 5.79 ppm (d, 1H, J = 11.41 Hz), 6.02-6.12 ppm (d, 1H, J = 11.38 Hz, of t, J = 7.37 Hz). EIMS *mlz:* M⁺ 217

### EXAMPLE IX

The MSG-IMP mixture (0.01% MSG and 0.005% IMP), an umami composition, was combined with Formulas II and III at different concentrations and evaluated by a trained sensory panel. Sensory evaluation was reported in the following:

| **Composition** | **Formula Concentration** | **Flavor Profile** |
|---|---|---|
| MSG-IMP | 0 | Heavy umami, full profile, slight sweetness of MSG perceived, enhanced umami in the back of mouth/throat |
| MSG-IMP/Formula II | 500 ppb | Increased mouth-feel, savory |
| | 1 ppm | Increased umami flavor and less greasy notes |
| | 5 ppm | Strong umami enhancement |
| MSG-IMP/Formula III | 500 ppb | Increased mouthfullness, delocalized umami |
| | 1 ppm | Increased mouthfullness, delocalized umami |

Formulas II and III enhanced the umami flavor of the MSG-IMP mixture.

## Claims

1. A compound selected from the group consisting of (E)-N-(2-methylbutyl)nona-2-en-6,8-diynamide and (Z)-N-(2-methylbutyl)nona-2-en-6,8-diynamide.

2. A composition comprising a compound selected from the group consisting of (E)-N-(2-methylbutyl)nona-2-en-6,8-diynamide and (Z)-N-(2-methylbutyl)nona-2-en-6,8-diynamide, and an umami compound.

3. A material selected from the group consisting of foodstuff, a chewing gum, a dental or oral hygiene product, and a medicinal product comprising the composition of claim 2, or of any of claims 4 to 10.

4. The composition of claim 2, wherein the umami compound is selected from the group consisting of monosodium glutamate, adenosine 5'-(trihydrogen diphosphate), 5'-cytidylic acid, 5'-uridylic acid, 5'-adenylic acid, 5'-guanylic acid, 5'-inosinic acid, 5'-guanylic acid disodium salt, 5'-inosinic acid disodium salt and a mixture thereof.

5. The composition of claim 4, wherein the umami compound is monosodium glutamate.

6. The composition of claim 4, wherein the umami compound is a mixture of monosodium glutamate and 5'-inosinic acid.

7. The composition of claim 4, or of any of claims 3 to 6, wherein the compound and the umami compound have a weight ratio of at least about 1:20,000.

8. The composition of claim 4, or of any of claims 3 to 6, wherein the compound and the umami compound have a weight ratio of from about 1:2,000 to about 1:2.

9. The composition of claim 4, or of any of claims 3 to 6, wherein the compound and the umami compound have a weight ratio of from about 1:1,000 to about 1:10.

10. The composition of claim 4, or of any of claims 3 to 6, wherein the compound and the umami compound have a weight ratio of from about 1:500 to about 1:20.

11. A process of augmenting, enhancing or imparting a taste to a material selected from the group consisting of foodstuff, a chewing gum, a dental or oral hygiene product, and a medicinal product comprising the step of incorporating an olfactory effective amount of a compound selected from the group consisting of (E)-N-(2-methylbutyl)nona-2-en-6,8-diynamide and (Z)-N-(2-methylbutyl)nona-2-en-6,8-diynamide and an umami compound.

12. The process of claim 11, wherein the umami compound is selected from the group consisting of monosodium glutamate, adenosine 5'-(trihydrogen diphosphate), 5'-cytidylic acid, 5'-uridylic acid, 5'-adenylic acid, 5'-guanylic acid, 5'-inosinic acid, 5'-guanylic acid disodium salt, 5'-inosinic acid disodium salt and a mixture thereof.

13. The process of claim 12, wherein the umami compound is monosodium glutamate.

14. The process of claim 12, wherein the umami compound is a mixture of monosodium glutamate and 5'-inosinic acid.

15. The process of claim 11, or of any of claims 12 to 14, wherein the compound and the umami compound have a weight ratio of at least about 1:20,000, or wherein the compound and the umami compound have a weight ratio of from about 1:2,000 to about 1:2, or wherein the compound and the umami compound have a weight ratio of from about 1:1,000 to about 1:10, or wherein the compound and the umami compound have a weight ratio of from about 1:500 to about 1:20.

## Patentansprüche

1. Verbindung ausgewählt aus der Gruppe bestehend aus (E)-N-(2-methylbutyl)non-2-en-6,8-diynamid und (Z)-N-(2-methylbutyl)non-2-en-6,8-diynamid.

2. Zusammensetzung, beinhaltend eine Verbindung ausgewählt aus der Gruppe bestehend aus (E)-N-(2-methylbutyl)non-2-en-6,8-diynamid und (Z)-N-(2-methylbutyl)non-2-en-6,8-diynamid und eine Umami-Verbindung.

3. Material ausgewählt aus der Gruppe bestehend aus Nährmittel, einem Kaugummi, einem Zahn- oder Mundhygieneprodukt und einem Arzneiprodukt, beinhaltend die Zusammensetzung gemäß Anspruch 2 oder gemäß einem der Ansprüche 4 bis 10.

4. Zusammensetzung gemäß Anspruch 2, wobei die Umami-Verbindung ausgewählt ist aus der Gruppe bestehend aus Mononatriumglutamat, Adenosin-5'-(Triwasserstoff-Diphosphat), 5'-Cytidylsäure, 5'-Uridylsäure, 5'-Adenylsäure, 5'-Guanylsäure, 5'-Inosinsäure, 5'-Guanylsäure-Dinatriumsalz, 5'-Inosinsäure-Dinatriumsalz und einer Mischung von diesen.

5. Zusammensetzung gemäß Anspruch 4, wobei die Umami-Verbindung Mononatriumglutamat ist.

6. Zusammensetzung gemäß Anspruch 4, wobei die Umami-Verbindung eine Mischung aus Mononatriumglutamat und 5'-Inosinsäure ist.

7. Zusammensetzung gemäß Anspruch 4 oder gemäß einem der Ansprüche 3 bis 6, wobei die Verbindung und die Umami-Verbindung ein Gewichtsverhältnis von mindestens etwa 1:20000 besitzen.

8. Zusammensetzung gemäß Anspruch 4 oder gemäß einem der Ansprüche 3 bis 6, wobei die Verbindung und die Umami-Verbindung ein Gewichtsverhältnis von etwa 1:2000 bis etwa 1:2 besitzen.

9. Zusammensetzung gemäß Anspruch 4 oder gemäß einem der Ansprüche 3 bis 6, wobei die Verbindung und die Umami-Verbindung ein Gewichtsverhältnis von etwa 1:1000 bis etwa 1:10 besitzen.

10. Zusammensetzung gemäß Anspruch 4 oder gemäß einem der Ansprüche 3 bis 6, wobei die Verbindung und die Umami-Verbindung ein Gewichtsverhältnis von etwa 1:1500 bis etwa 1:20 besitzen.

11. Verfahren zum Verstärken, Verbessern oder Vermitteln eines Geschmacks für ein Material ausgewählt aus der Gruppe bestehend aus Nährmittel, einem Kaugummi, einem Zahn- oder Mundhygieneprodukt und einem Arzneiprodukt, beinhaltend den Schritt des Inkorporierens einer olfaktorisch effektiven Menge einer Verbindung ausgewählt aus der Gruppe bestehend aus (E)-N-(2-methylbutyl)non-2-en-6,8-diynamid und (Z)-N-(2-methylbutyl)non-2-en-6,8-diynamid und einer Umami-Verbindung

12. Verfahren gemäß Anspruch 11, wobei die Umami-Verbindung ausgewählt ist aus der Gruppe bestehend aus Mononatriumglutamat, Adenosin-5'-(Triwasserstoff-Diphosphat), 5'-Cytidylsäure, 5'-Uridylsäure, 5'-Adenylsäure, 5'-Guanylsäure, 5'-Inosinsäure, 5'-Guanylsäure-Dinatriumsalz, 5'-Inosinsäure-Dinatriumsalz und einer Mischung von diesen.

13. Verfahren gemäß Anspruch 12, wobei die Umami-Verbindung Mononatriumglutamat ist.

14. Verfahren gemäß Anspruch 12, wobei die Umami-Verbindung eine Mischung aus Mononatriumglutamat und 5'-Inosinsäure ist.

15. Verfahren gemäß Anspruch 11 oder gemäß einem der Ansprüche 12 bis 14, wobei die Verbindung und die Umami-Verbindung ein Gewichtsverhältnis von mindestens etwa 1:20000 besitzen, oder wobei die Verbindung und die Umami-Verbindung ein Gewichtsverhältnis von etwa 1:2000 bis etwa 1:2 besitzen, oder wobei die Verbindung und die Umami-Verbindung ein Gewichtsverhältnis von etwa 1:1000 bis etwa 1:10 besitzen, oder wobei die Verbindung und die Umami-Verbindung ein Gewichtsverhältnis von etwa 1:500 bis etwa 1:20 besitzen.

## Revendications

1. Un composé sélectionné dans le groupe se composant de (E)-N-(2-méthylbutyle)nona-2-en-6,8-diynamide et de (Z)-N-(2-méthylbutyle)nona-2-en-6,8-diynamide.

2. Une composition contenant un composé sélectionné dans le groupe se composant de (E)-N-(2-méthylbutyle)nona-2-en-6,8-diynamide et de (Z)-N-(2-méthylbutyle)nona-2-en-6,8-diynamide, et d'un composé umami.

3. Un matériau sélectionné dans le groupe se composant d'aliments, d'une pâte à mâcher, d'un produit d'hygiène buccale ou dentaire et d'un produit médicinal contenant la composition selon la Revendication 2, ou selon l'une quelconque des Revendications 4 à 10.

4. La composition selon la Revendication 2, où le composé umami est sélectionné dans le groupe se composant de glutamate monosodique, d'adénosine 5'-(trihydrogène diphosphate), d'acide 5'-cytidylique, d'acide 5'-uridylique, d'acide 5'-adénylique, d'acide 5'-guanylique, d'acide 5'-inosinique, de sel disodique d'acide 5'-guanylique, de sel disodique d'acide 5'-inosinique, et d'un mélange de ceux-ci.

5. La composition selon la Revendication 4, où le composé umami est glutamate monosodique.

6. La composition selon la Revendication 4, où le composé umami est un mélange de glutamate monosodique et d'acide 5'-inosinique.

7. La composition selon la Revendication 4, ou selon l'une quelconque des Revendications 3 à 6, où le composé et le composé umami présentent un rapport de poids d'au moins environ 1:20 000.

8. La composition selon la Revendication 4, ou selon l'une quelconque des Revendications 3 à 6, où le composé et le composé umami présentent un rapport de poids d'environ 1:2 000 à environ 1:2.

9. La composition selon la Revendication 4, ou selon l'une quelconque des Revendications 3 à 6, où le composé et le composé umami présentent un rapport de poids d'environ 1:1 000 à environ 1:10.

10. La composition selon la Revendication 4, ou selon l'une quelconque des Revendications 3 à 6, où le composé et le composé umami présentent un rapport de poids d'environ 1:500 à environ 1:20.

11. Un processus d'augmentation, d'amélioration ou d'attribution d'un goût à un matériau sélectionné dans le groupe se composant d'aliments, d'une pâte à mâcher, d'un produit d'hygiène buccale ou dentaire et d'un produit médicinal comprenant l'opération d'incorporation d'une quantité efficace sur le plan olfactif d'un composé sélectionné dans le groupe se composant de (E)-N-(2-méthylbutyle)nona-2-en-6,8-diynamide et de (Z)-N-(2-méthylbutyle)nona-2-en-6,8-diynamide et d'un composé umami.

12. Le processus selon la Revendication 11, où le composé umami est sélectionné dans le groupe se composant de glutamate monosodique, adénosine 5'-(trihydrogène diphosphate), d'acide 5'-cytidylique, d'acide 5'-uridylique, d'acide 5'-adénylique, d'acide 5'-guanylique, d'acide 5'-inosinique, de sel disodique d'acide 5'-guanylique, de sel disodique d'acide 5'-inosinique, et d'un mélange de ceux-ci.

13. Le processus selon la Revendication 12, où le composé umami est glutamate monosodique.

14. Le processus selon la Revendication 12, où le composé umami est un mélange de glutamate monosodique et d'acide 5'-inosinique.

15. Le processus selon la Revendication 11, ou selon l'une quelconque des Revendications 12 à 14, où le composé et le composé umami présentent un rapport de poids d'au moins environ 1:20 000, ou où le composé et le composé umami présentent un rapport de poids d'environ 1:2 000 à environ 1:2, ou où le composé et le composé umami présentent un rapport de poids d'environ 1:1 000 à environ 1:10, ou où le composé et le composé umami présentent un rapport de poids d'environ 1:500 à environ 1:20.
